# NEW EUROPEAN PATENT SPECIFICATION

(11) **EP 0 620 206 B2**
(45) Date of publication and mention of the opposition decision: **17.05.2000**
(45) Mention of the grant of the patent: 24.09.1997
(21) Application number: 94105788.7
(22) Date of filing: 14.04.1994
(51) Int. Cl.: C07C 57/04, C07C 69/54, C07C 51/50, C07C 67/62

(54) **Method for inhibiting polymerization of (meth)acrylic acid and esters thereof**
Verfahren zur Polymerisationsinhibierung von (Meth-) Acrylsäure und ihrer Ester
Procédé d'inhibition de la polymérisation d'acide (méth-)acrylique et de ses esters

(30) Priority: 15.04.1993 JP 8890893
(43) Date of publication of application: 19.10.1994
(73) Proprietor: NIPPON SHOKUBAI CO., LTD., Chuo-ku, Osaka-shi, Osaka-fu 541 (JP)
(72) Inventor: Sakamoto, Kazuhiko, Himeji-shi, Hyogo 671-22 (JP); Takeda, Takahiro, Himeji-shi, Hyogo 671-12 (JP); Ueoka, Masatoshi, Himeji-shi, Hyogo 670 (JP); Akazawa, Yoji, Himeji-shi, Hyogo 672 (JP); Baba, Masao, Himeji-shi, Hyogo 671-11 (JP)
(74) Representative: VOSSIUS & PARTNER

(56) References cited:
- EP-A- 0 178 168
- EP-A- 0 522 709
- GB-A- 1 265 419
- US-A- 3 794 567
- Chemical Journal of Chinese University, vol. 4, no. 2, 1983, translation, pp.1-7

## Description

The present invention relates to a method for inhibiting the polymerization of (meth)acrylic acid monomers and (meth)acrylate monomers (referred to as (meth)acrylic acid and esters thereof hereinafter) that will otherwise take place during their production. In the present invention the term "(meth)acrylic acid" means "acrylic acid or methacrylic acid".

It is well-known that (meth)acrylic acid and esters thereof have a strong tendency to spontaneous polymerization by light or heat. Hence, it is common practice to add one inhibitor or more in combination to inhibit polymerization during their storage.

An example of the inhibitors which have so far been tried is N-oxyl compounds, such as di-tert-butyl nitroxide and 2,2,6,6-tetramethyl-4-hydroxypiperidinooxyl(2,2,6,6-tetramethyl-4-hydroxypiperidine-1-oxyl), as disclosed in British Patent No. 1,127,127. According to the disclosure, they are more effective than known inhibitors such as hydroquinone, phenothiazine, and cupric chloride, when they are used alone. The N-oxyl compound is also used as an inhibitor in the production of methacrylic acid from methacrolein by the aid of an oxygen-containing gas in an organic solvent. It includes 2,2,6,6-tetramethyl-4-hydroxypiperidinooxyl and 2,2,6,6-tetramethylpiperidinooxyl as disclosed in U.S. Patent No. 4,127,603. It also includes 2,2,5,5-tetramethyl-3-oxopyrrolidinooxyl and 2,2,6,6-tetramethyl-4-acetoxypiperidinooxyl as disclosed in Japanese Patent Publication No. 46496/1983. In addition, it is shown in Chinese Patent Laid-Open No. 1,052,847A that 2,2,6,6-tetramethyl-4-hydroxypiperidinooxyl effectively inhibits the polymerization of acrylic acid and acrylate when used alone or in combination with hydroquinone and that this(or these) inhibitor(s) is(are) more effective than copper dibutyldithiocarbamate and hydroquinone used in combination.

Contrary to the foregoing, the present inventors have found that the N-oxyl compound used alone or in combination with hydroquinone does not work satisfactorily under specific conditions. In other words, the above-mentioned inhibitor used in an ordinary amount does not work in the production of (meth)acrylic acid by catalytic gas phase reaction, because polymerization of (meth)acrylic acid easily occurs while it is separated by azeotropic distillation from its aqueous solution containing acetic acid and aldehydes. Thus, there was a problem of the formation of popcorn polymers and sticky polymers in the distillation column during distillation, which prevents the continuous operation of the plant for a long period of time. Since large amount of inhibitor was required to obtain effective inhibition, this process was not practical from a commercial standpoint.

It is an object of the present invention to provide a method for inhibiting the polymerization of (meth)acrylic acid and esters thereof with a small amount of inhibitor in the above-mentioned process.

The present invention provides a method for inhibiting the polymerization of (meth)acrylic acid and esters thereof by using as the inhibitor an admixture of (A) at least one N-oxyl compound selected from 2,2,6,6-tetramethylpiperidinooxyl, 4-hydroxy-2,2,6,6-tetramethylpiperidinooxyl, and 4,4',4''-tris-(2,2,6,6-tetramethylpiperidinooxyl)phosphite, (B) at least one phenol compound selected from hydroquinone and methoquinone (p-methoxyphenol), and (C) at least one phenothiazine compound.

A method wherein said inhibitor-admixture is added to the (meth)acrylic acid or esters thereof in destillation processes is within the present invention.

The essential prerequisite of the present invention resides in using as the inhibitor an admixture of (A), (B) and (C) which are as defined above. The present inventors have found that only when used together do these compounds provide a remarkable synergistic effect for inhibiting the polymerization of (meth)acrylic acid and esters thereof which they incompletely provide when used alone or in pairs. The present invention is based on this finding.

In the present invention, the at least one N-oxyl compound is selected from 2,2,6,6-tetramethylpiperidinooxyl, 4-hydroxy-2,2,6,6,-tetramethylpiperidinooxyl, and 4,4',4''-tris-(2,2,6,6-tetramethylpiperidinooxyl)phosphite, alone or in combination with one another. The use of 2,2,6,6-tetramethylpiperidinooxyl or 4-hydroxy-2,2,6,6,-tetramethylpiperidinooxyl is desirable because these compounds are not associated with metal ions. Thus, the plant device is free from corrosion and waste treatment becomes easy.

The phenol compound used in the present invention includes hydroquinone and methoquinone. Methoquinone, or p-methoxyphenol is preferably used because the inhibiting effect of methoquinone is superior to hydroquinone when used in combination with the at least one N-oxyl compound and the at least one phenothiazine compound. Hydroquinone and methoquinone may be used alone or in combination with each other.

The at least one phenothiazine compound used in the present invention includes, for example, phenothiazine, bis-(α-methylbenzyl)phenothiazine, 3,7-dioctylphenothiazine, and bis-(α-dimethylbenzyl)phenothiazine. Phenothiazine is preferred. They may be used alone or in combination with one another.

According to the present invention, the above-mentioned inhibitor-admixture of (A), (B) and (C) produces a pronounced inhibiting effect. If necessary, said admixture may also be used in combination with molecular oxygen to enhance the inhibiting effect and to help provide for a continuous operation of the plant for a long period of time.

The inhibiting method of the present invention is applied to (meth)acrylic acid and esters thereof which are particularly liable to autopolymerization. Examples of the acrylic ester include methyl acrylate, ethyl acrylate, butyl acrylate, 2-ethylhexyl acrylate, 2-hydroxyethyl acrylate, and 2-hydroxypropyl acrylate. Examples of the methacrylic ester include methyl methacrylate, butyl methacrylate, 2-hydroxyethyl methacrylate, and 2-hydroxypropyl methacrylate.

According to the inhibiting method of the present invention, these three types of inhibitor components are added to (meth)acrylic acid in distillation processes, such as those which employ a rectification column, a separation column for separation from solvent, a separation column for separation from low-boiling fractions such as acetic acid, and a stripper for separation of low-boiling acrolein and methacrolein, in the case where (meth)acrylic acid is produced by the catalytic gas phase reaction.

The method of the present invention comprises adding these three types of inhibitor components during the above-mentioned processes. The inhibitor components may be added in solid or powder form or in the form of an aqueous solution or solvent solution. The three types of inhibitor components may be added all at once (in the form of solution) or one after another. If the inhibitor is to be added during the distillation process in the production of (meth)acrylic acid, it may be dissolved in the feed or reflux.

The amount of the three inhibitor components to be used is not specifically limited, depending on the operation conditions. Preferably, the total amount is 2-1000 ppm, (by weight) based on the amount of evaporated monomer (or (meth)acrylic acid and esters thereof). The preferred amount of the individual inhibitor components (A), (B) and (C) based on the amount of evaporated monomer is as follows:
(A) N-oxyl compound(s): 0.1-100 ppm
(B) Phenol compound(s): 1-500 ppm
(C) Phenothiazine compound(s): 1-500 ppm

(The second and third inhibitor components are preferably used in the same amount).

The amount of evaporated monomer means the total amount of monomer vapor which is boiled up from the bottom of the column in proportion to the amount of heat fed to the reboiler. This factor may be used to determine the amount of the inhibitor components to be added.

The optional molecular oxygen may be added to (meth)acrylic acid and esters thereof directly by bubbling or indirectly by dissolution in solvents. Bubbling may be easily accomplished by introducing oxygen gas into the distillation column or stripper from their bottom and/or from the reboiler. The amount of molecular oxygen is preferably 0.1-1.0 vol% of the amount of evaporated monomer.

The method for inhibiting polymerization of the present invention has been described above. It consists of using the three types of inhibitor components - (A) at least one N-oxyl compound, (B) at least one phenol compound, and (C) at least one phenothiazine compound. The combined use of (A), (B) and (C) provides superior inhibiting effect to use alone or in pairs. It has now become possible to prevent (meth)acrylic acid and esters thereof from polymerizing even under conditions which promote their polymerization. Thus the method of the present invention provides for the uninterrupted operation of the plant for a long period and stable transportation and storage of (meth)acrylic acid and esters thereof.

### EXAMPLES

To further illustrate the invention the following examples are given. (Unit ppm in the following examples and comparative examples is by weight)

### Example 1

Pure acrylic acid was prepared from commercial one by distillation to remove inhibitors. 2ml each of the pure acrylic acid (placed in a test tube) was incorporated with inhibitors in different amounts as shown in Table 1. (Experiments Nos. 1-11 are for comparison.) The test tube was kept under reduced pressure and immersed in an oil bath at 100°C.

Induction period of polymerzation was measured by visual inspection. The results are shown in Table 1. Compounds are abbreviated as follows hereinafter.
- TEMPO :: 2,2,6,6-tetramethylpiperidinooxyl
- 4H-TEMPO :: 4-hydroxy-2,2,6,6-tetramethylpiperidinooxyl
- TRIS-TEMPO :: 4,4',4''-tris-(2,2,6,6-tetramethylpiperidinooxyl)phosphite
- DTBNO :: di-tert-butyl nitroxide
- HQ :: hydroquinone
- MQ :: methoquinone(=p-methoxyphenol)
- BQ :: benzoquinone
- PTZ :: phenothiazine

**Table 1**

| Run No. | Inhibitors (ppm by weight) | | | | | | Induction period (min) |
|---|---|---|---|---|---|---|---|
| | N-oxyl compd. | | HQ | MQ | BQ | PTZ | |
| 1 | 4H-TEMPO | 1 | 0 | 0 | 0 | 0 | 8 |
| 2 | 4H-TEMPO | 0 | 13 | 0 | 0 | 0 | 3 |
| 3 | 4H-TEMPO | 0 | 0 | 13 | 0 | 0 | 6 |
| 4 | 4H-TEMPO | 0 | 0 | 0 | 0 | 13 | 20 |
| 5 | 4H-TEMPO | 1 | 13 | 0 | 0 | 0 | 10 |
| 6 | 4H-TEMPO | 1 | 0 | 13 | 0 | 0 | 15 |
| 7 | 4H-TEMPO | 1 | 0 | 0 | 0 | 13 | 25 |
| 8 | 4H-TEMPO | 0 | 13 | 0 | 0 | 13 | 21 |
| 9 | 4H-TEMPO | 1 | 0 | 0 | 6.5 | 6.5 | 24 |
| 10 | DTBNO | 1 | 6.5 | 0 | 0 | 6.5 | 26 |
| 11 | DTBNO | 1 | 0 | 6.5 | 0 | 6.5 | 28 |
| 12 | 4H-TEMPO | 1 | 6.5 | 0 | 0 | 6.5 | 38 |
| 13 | 4H-TEMPO | 1 | 0 | 6.5 | 0 | 6.5 | 57 |
| 14 | 4H-TEMPO | 0.5 | 3.3 | 0 | 0 | 3.3 | 31 |
| 15 | 4H-TEMPO | 0.5 | 0 | 3.3 | 0 | 3.3 | 47 |
| 16 | TEMPO | 1 | 6.5 | 0 | 0 | 6.5 | 40 |
| 17 | TRIS-TEMPO | 1 | 6.5 | 0 | 0 | 6.5 | 35 |

From Table 1 it is clear that the inhibiting effect (expressed in terms of induction period) is better in experiments Nos. 12-17 (pertaining to the present invention) than in experiments Nos. 1-8 (for comparison) in which the inhibitors were used alone or in pairs. It is to be noted that experiment No. 14-15 gave better results than experiments Nos. 5-8 although the total amount of the inhibitors is less. This illustrates the importance of using the three inhibitors together. Further, the comparative experiment No.9 using benzoquinone instead of hydroquinone or methoquinone, and the experiment Nos. 10-11 using DTBNO instead of 4H-TEMPO showed shorter induction period than experiments Nos. 12-13. Because benzoquinone and DTBNO are not specified in the present invention, the comparative experiments Nos.9-11 gave inferior results even using three inhibitors.

### Example 2

The inhibiting effect was evaluated in azeotropic separation of acrylic acid from its aqueous solution (containing 30 wt% water and 2.5 wt% acetic acid) resulting from the catalytic gas phase reaction of propylene. The azeotropic separation was carried out using a packed column with a feed pipe in the middle and a condenser at the top and also using methyl isobutyl ketone as reflux, with the pressure being 21.3 kPa (160 mmHg) and the temperature being 49 °C at the top and 97 °C at the bottom. Each of the inhibitors shown in Table 2 was added to the reflux (which was fed to the column) and oxygen gas was fed to the bottom of the column. The amounts of the inhibitors and oxygen are based on the amount of evaporated acrylic acid. It was found that the bottom product in the steady state was comprised of 97 wt% acrylic acid and 0.5 wt% acetic acid, with the remainder being 2.5 wt%. After continued operation for 8 hours, with the reflux being recycled as such, the amount of polymers formed in the column was determined through suction drying to obtain constant weight. The results are shown in Table 2.

**Table 2**

| Run No. | Inhibitors (ppm) | | | Amount of oxygen (vol%) | Amount of polymers formed (g) |
|---|---|---|---|---|---|
| | 4H-TEMPO | HQ | PTZ | | |
| 18 | 3.8 | 0 | 0 | 0.21 | 9.0 |
| 19 | 1 | 6.7 | 0 | 0.21 | 2.9 |
| 20 | 0.5 | 3.3 | 0 | 0.21 | 33.2 |
| 21 | 1 | 0 | 6.7 | 0.21 | 3.0 |
| 22 | 0.5 | 0 | 3.3 | 0.21 | 25.3 |
| 23 | 1 | 3.3 | 3.3 | 0.21 | 0.6 |
| 24 | 0.5 | 1.7 | 1.7 | 0.21 | 1.2 |

From Table 2 it is clear that the amount of polymers formed was less in experiments Nos. 23 and 24 (pertaining to present invention) than in experiments Nos. 18 to 22 (for comparison) in which the three inhibitors were not used together.

### Example 3

Pure methacrylic acid was prepared from commercial one by distillation to remove inhibitors. 2ml each of the pure methacrylic acid (placed in a test tube) was incorporated with inhibitors in different amounts as shown in Table 3. (Experiments Nos. 25-27 are for comparison.) The test tube was kept under reduced pressure and immersed in an oil bath at 130°C.

Induction period of polymerization was measured by visual inspection. The results are shown in Table 3.

**Table 3**

| Run No. | Inhibitors (ppm by weight) | | | | Induction period (min) |
|---|---|---|---|---|---|
| | 4H-TEMPO | HQ | MQ | PTZ | |
| 25 | 1 | 13 | 0 | 0 | 20 |
| 26 | 1 | 0 | 13 | 0 | 23 |
| 27 | 1 | 0 | 0 | 13 | 27 |
| 28 | 1 | 6.5 | 0 | 6.5 | 45 |
| 29 | 0.5 | 3.3 | 0 | 3.3 | 38 |
| 30 | 1 | 0 | 6.5 | 6.5 | 68 |
| 31 | 0.5 | 0 | 3.3 | 3.3 | 57 |

From Table 3 it is clear that the inhibiting effect (expressed in terms of induction period) is better in experiments Nos. 28-31 (pertaining to the present invention) than in experiments Nos. 25-27 (for comparison) in which the inhibitors were used in pairs. It is to be noted that experiment No. 29 and 31 gave better results than experiments Nos. 25-27 although the total amount of the inhibitors is less.

### Example 4

Four pure acrylates (methyl acrylate (AM), ethyl acrylate (AE), butyl acrylate (AB), and octyl acrylate (AO)) were prepared from commercial ones by distillation to remove inhibitors. 30ml each of the pure acrylate (placed in a test tube) was incorporated with inhibitors in different amounts as shown in Table 4. The test tube was kept under reduced pressure and immersed in an oil bath at 70 °C for AM, 90 °C for AE, and 120 °C for AB and AO. Induction period of polymerization was measured by heat generation. The results are shown in Table 4.

**Table 4**

| Run No. | Inhibitors (ppm by weight) | | | | Induction period (hr) | | | |
|---|---|---|---|---|---|---|---|---|
| | 4H-TEMPO | HQ | MQ | PTZ | AM | AE | AB | AO |
| 32 | 1 | 13 | 0 | 0 | 11.0 | 10.5 | 7.5 | 20.5 |
| 33 | 1 | 0 | 13 | 0 | 54.0 | 56.0 | 38.0 | 85.0 |
| 34 | 1 | 0 | 0 | 13 | 77.5 | 79.0 | 57.0 | 139 |
| 35 | 1 | 6.5 | 0 | 6.5 | 141 | 135 | 89.5 | 233 |
| 36 | 0.5 | 3.3 | 0 | 3.3 | 106 | 117 | 68.0 | 178 |
| 37 | 1 | 0 | 6.5 | 6.5 | 197 | 189 | 125 | 326 |
| 38 | 0.5 | 0 | 3.3 | 3.3 | 148 | 164 | 95.0 | 249 |

From Table 4 it is clear that the induction period of all acrylates in experiments Nos. 35-38 (pertaining to the present invention) is longer than in experiments Nos. 32-34 (for comparison) in which the inhibitors were used in pairs.

### Example 5

Two pure methacrylates (methyl methacrylate (MMA) and butyl methacrylate (BMA)) were prepared from commercial ones by distillation to remove inhibitors. 30ml each of the pure methacrylates (placed in a test tube) was incorporated with inhibitors in different amounts as shown in Table 5. The test tube was kept under reduced pressure and immersed in an oil bath at 90°C for MMA and 120°C for BMA.

Induction period of polymerization was measured in the same manner as in Example 4. The results are shown in Table 5.

**Table 5**

| Run No. | Inhibitors (ppm by weight) | | | | Indct.period (hr) | |
|---|---|---|---|---|---|---|
| | 4H-TEMPO | HQ | MQ | PTZ | MMA | BMA |
| 39 | 1 | 13 | 0 | 0 | 33.5 | 15.0 |
| 40 | 1 | 0 | 13 | 0 | 21.0 | 12.0 |
| 41 | 1 | 0 | 0 | 13 | 16.0 | 9.5 |
| 42 | 1 | 6.5 | 0 | 6.5 | 65.0 | 29.5 |
| 43 | 0.5 | 3.3 | 0 | 3.3 | 43.0 | 21.0 |
| 44 | 1 | 0 | 6.5 | 6.5 | 84.5 | 38.0 |
| 45 | 0.5 | 0 | 3.3 | 3.3 | 56.0 | 27.0 |

From Table 5 it is clear that the induction period in experiments Nos. 42-45 (pertaining to the present invention) is longer than in experiments Nos. 39-41 (for comparison) in which the inhibitors were used in pairs.

### Example 6

Four pure (meth) acrylates (listed below) were prepared from commercial ones by distillation to remove inhibitors. 30ml each of the pure (meth) acrylates (placed in a test tube) was incorporated with inhibitors in different amounts as shown in Table 6. The test tube was kept under reduced pressure and immersed in an oil bath at 100°C.

Induction period of polymerization was measured by heat generation. The results are shown in Table 6.
- HEA :: 2-hydroxyethyl acrylate
- HPA :: 2-hydroxypropyl acrylate
- HEMA :: 2-hydroxyethyl methacrylate
- HPMA :: 2-hydroxypropyl methacrylate

**Table 6**

| Run No. | Inhibitors (ppm by weight) | | | | Induction period (hr) | | | |
|---|---|---|---|---|---|---|---|---|
| | 4H-TEMPO | HQ | MQ | PTZ | HEA | HPA | HEMA | HPMA |
| 46 | 1 | 13 | 0 | 0 | 29.5 | 91.0 | 72.0 | 37.5 |
| 47 | 1 | 0 | 13 | 0 | 33.0 | 96.0 | 78.0 | 41.0 |
| 48 | 1 | 0 | 0 | 13 | 35.0 | 100 | 84.5 | 45.5 |
| 49 | 1 | 6.5 | 0 | 6.5 | 57.0 | 175 | 145 | 71.0 |
| 50 | 0.5 | 3.3 | 0 | 3.3 | 49.5 | 133.5 | 113.5 | 59.5 |
| 51 | 1 | 0 | 6.5 | 6.5 | 74.0 | 228 | 189 | 92.0 |
| 52 | 0.5 | 0 | 3.3 | 3.3 | 64.0 | 174 | 148 | 77.0 |

From Table 6 it is clear that the inhibiting effect in experiments Nos. 49-52 (pertaining to the present invention) is better than in experiments Nos. 46-48 (for comparison).

### Example 7

The inhibiting effect was evaluated in azeotropic separation of acrylic acid from its aqueous solution (containing 30 wt% water and 2.5 wt% acetic acid) resulting from the catalytic gas phase reaction of propylene. The azeotropic separation was carried out using a distillation column (105 mm in inside diameter) equipped with 50 stainless steel sieve trays at intervals of 147 mm, with a feed pipe in the middle and a condenser at the top and also using methyl isobutyl ketone as reflux, under the following conditions.
Temperature at the column top: 46°C
Temperature at the column bottom: 97°C
Pressure at the column top: 14,66 kPa (110 mmHg)
Pressure at the column bottom: 20,26 kPa (152 mmHg)
Amount of feed at the 37th tray: 10.58 L/h
Amount of reflux: 19.54 L/h
Amount of discharge from the bottom: 7.05 L/h
Amount of water phase distilled: 3.94 L/h
Amount of oil phase distilled: 19.74 L/h
R/D = 0.92 (in molar ratio)

Each of the following inhibitors was added to methyl isobutyl ketone (which was fed to the top of the column) and oxygen gas was fed to the bottom of the column in an amount of 0.3 vol%.
4-hydroxyl-2,2,6,6-tetramethylpiperidinooxyl (4H-TEMPO), 30 ppm
hydroquinone (HQ), 100 ppm
phenothiazine (PTZ), 100 ppm

The amounts of the inhibitors and oxygen are based on the amount of evaporated acrylic acid.

It was found that the bottom product in the steady state was comprised of 97.2 wt% acrylic acid and 0.5 wt% acetic acid, with the remainder being 2.3 wt%, the water phase distilled was composed of 90.8 wt% water, 0.4 wt% acrylic acid, 6.5 wt% acetic acid, and 2.3 wt% methyl isobutyl ketone, and the oil phase distilled was composed of 86.7 wt% methyl isobutyl ketone, 3.9 wt% water, 1.4 wt% acrylic acid, 6.5 wt% acetic acid, with the remainder being 1.5 wt%. The oil phase distilled was recycled as the reflux. The inhibiting effect was evaluated by observing the pressure drop in the column or flooding, or by disassembling the column. It was possible to carry out continued operation for 14 days in a stable manner. The distillation column was disassembled for inspection after stopping operation. The formation of polymers was not found at all.

### Comparative Example 1

The same procedure as in Example 7 was repeated except that PTZ was omitted and the amount of HQ was increased to 200 ppm (with the total amount of the inhibitors remaining unchanged). After 2 days, it became impossible to continue operation due to pressure drop in the column. Inspection by disassembling the column revealed the formation of popcorn polymers. The result indicates that the two inhibitors alone (N-oxyl compound and phenol compound) are not enough to provide the desired inhibiting effect.

### Comparative Example 2

The same procedure as in Example 7 was repeated except that HQ was omitted and the amount of PTZ was increased to 200 ppm (with the total amount of the inhibitors remaining unchanged). After 4 days, it became impossible to continue operation due to pressure drop in the column. Inspection by disassembling the column revealed the formation of popcorn polymers. The result indicates that the two inhibitors alone (N-oxyl compound and phenothiazine) are not enough to provide the desired inhibiting effect.

## Claims

1. A method for inhibiting the polymerization of acrylic acid or methacrylic acid and esters thereof, by using as the inhibitor an admixture of (A) at least one N-oxyl compound selected from 2,2,6,6-tetramethylpiperidinooxyl, 4-hydroxy-2,2,6,6-tetramethylpiperidinooxyl, and 4,4',4''-tris-(2,2,6,6-tetramethylpiperidinooxyl)phosphite, (B) at least one phenol compound selected from hydroquinone and methoquinone, and (C) at least one phenothiazine compound.

2. A method for inhibiting polymerization as defined in claim 1, wherein said inhibitor is used in total amount of 21-1000 ppm (by weight) based on the amount of evaporated acrylic acid, methacrylic acid or esters thereof.

3. A method for inhibiting polymerization as defined in claim 1, wherein said inhibitor components (A), (B) and (C) are used in an amount of 0.1-100 ppm, 1-500 ppm, and 1-500 ppm (by weight), respectively, based on the amount of evaporated acrylic acid, methacrylic acid or esters thereof.

4. A method for inhibiting polymerization as defined in claims 1 to 3, wherein said inhibitor-admixture is added to acrylic acid, methacrylic acid or esters thereof in destillation processes.

## Patentansprüche

1. Verfahren zur Inhibierung der Polymerisation von Acrylsäure oder Methacrylsäure und Estern davon unter Verwendung eines Gemisches aus (A) wenigstens einer N-Oxylverbindung, ausgewählt aus 2,2,6,6-Tetramethylpiperidinooxyl, 4-Hydroxy-2,2,6,6-tetramethylpiperidinooxyl und 4,4',4''-Tris-(2,2,6,6-tetramethylpiperidinooxyl)phosphit, (B) wenigstens einer Phenolverbindung, ausgewählt aus Hydrochinon und Methochinon, und (C) wenigstens einer Phenothiazinverbindung als Inhibitor.

2. Verfahren zur Inhibierung der Polymerisation nach Anspruch 1, wobei der Inhibitor in einer Gesamtmenge von 2-1000 ppm (in Gewicht), basierend auf der Menge an abgedampfter Acrylsäure, Methacrylsäure oder Estern davon, verwendet wird.

3. Verfahren zur Inhibierung der Polymerisation nach Anspruch 1, wobei die Inhibitorkomponenten (A), (B) und (C) in einer Menge von 0,1-100 ppm, 1-500 ppm beziehungsweise 1-500 ppm (in Gewicht), basierend auf der Menge an abgedampfter Acrylsäure, Methacrylsäure oder Estern davon, verwendet werden.

4. Verfahren zur Inhibierung der Polymerisation nach den Ansprüchen 1 bis 3, wobei das Inhibitorgemisch in Destillationsverfahren zu Acrylsäure, Methacrylsäure oder Estern davon gegeben wird.

## Revendications

1. Procédé d'inhibition de la polymérisation de l'acide acrylique ou de l'acide méthacrylique et de leurs esters, en utilisant, à titre d'inhibiteur, un mélange de (A) au moins un composé N-oxyle choisi parmi le 2,2,6,6-tétraméthylpipéridinooxyle, le 4-hydroxy-2,2,6,6-tétraméthylpipéridinooxyle, et le 4,4',4''-tris-(2,2,6,6-tétraméthylpipéridinooxyl)phosphite, (B) au moins un composé de phénol choisi parmi l'hydroquinone et la méthoquinone et (C) au, moins un composé de phénothiazine.

2. Procédé d'inhibition de la polymérisation selon la revendication 1, dans lequel ledit inhibiteur est utilisé en une quantité totale de 2-1000 ppm (en poids), sur la base de la quantité d'acide acrylique, d'acide méthacrylique, ou d'ester de ceux-ci évaporé.

3. Procédé d'inhibition de la polymérisation selon la revendication 1, dans lequel lesdits composés inhibiteurs (A), (B) et (C) sont utilisés en une quantité de 0,1-100 ppm, 1-500 ppm et 1-500 ppm (en poids), respectivement, sur la base de la quantité d'acide acrylique, d'acide méthacrylique, ou d'ester de ceux-ci évaporé.

4. Procédé d'inhibition de la polymérisation selon les revendications 1 à 3, dans lequel ledit mélange d'inhibiteurs est ajouté à l'acide acrylique, l'acide méthacrylique ou leurs esters dans des procédés de distillation.
